# EUROPEAN PATENT APPLICATION

(11) **EP 4 442 263 A1**
(43) Date of publication of application: **09.10.2024**
(21) Application number: 22901526.8
(22) Date of filing: 21.09.2022
(51) Int. Cl.: A61K 35/28, A61K 38/17, A61P 11/00

(54) **COMPOSITION FOR PREVENTING OR TREATING LUNG DISEASES CONTAINING CD47-OVEREXPRESSING MESENCHYMAL STEM CELLS**

(30) Priority: 01.12.2021 KR 20210170214
(71) Applicant: Medipost Co., Ltd., Bundang-gu, Seongnam-si Gyeonggi-do 13494 (KR)
(72) Inventor: YANG, Yun Sun, Seongnam-si Gyeonggi-do 13494 (KR); OH, Won Il, Seongnam-si Gyeonggi-do 13494 (KR); CHOI, Soo Jin, Seongnam-si Gyeonggi-do 13494 (KR); JIN, Hye Jin, Seongnam-si Gyeonggi-do 13494 (KR); BAE, Yun Kyung, Seongnam-si Gyeonggi-do 13494 (KR); LEE, Hyang Ju, Seongnam-si Gyeonggi-do 13494 (KR)
(74) Representative: Vossius & Partner Patentanwälte Rechtsanwälte mbB
(86) International application number: PCT/KR2022/014100
(87) International publication number: WO 2023/101166

(57) **Abstract**

Provided are a composition for use in preventing or treating lung disease comprising mesenchymal stem cells overexpressing CD47 as an active ingredient. The mesenchymal stem cells overexpressing CD47 show a high survival rate and ability to migrate to lung tissues *in vivo* even when they are not autologous, reduce the inflammatory response in lung tissues, and exhibit the effect of suppressing fibrosis, and therefore can be widely used for the prevention or treatment of various lung diseases associated with lung inflammation or fibrosis.

## Description

### Technical Field

The disclosure relates to a pharmaceutical composition for use in preventing or treating lung disease containing mesenchymal stem cells overexpressing CD47 as an active ingredient.

### Background Art

The lung is an essential organ responsible for breathing in the human body, and is an organ that obtains oxygen and excretes carbon dioxide through inhalation and exhalation of air, respectively. The lung functions to maintain vital phenomena, and in the lung, body fluid components are actively absorbed or discharged through epithelial cells, and in some cases, the lung can act as a delivery channel for inhaled drugs.

Asthma, a representative disease among these lung diseases, is a disease whose incidence has doubled over the past 20 years and affects 8-10% of the world's population today, and is mainly caused by chronic inflammation of the airways. Asthma is characterized by airway hyperresponsiveness (AHR) to non-specific stimuli and airway remodeling, the latter involving a dynamic process that leads to cellular changes in fibroblasts and myofibroblasts and structural changes in the airways. Types of asthma include bronchial asthma and cardiac asthma, and in general, asthma refers to bronchial asthma. Repeated episodes of asthma can lead to fibrosis and airway remodeling, which can result in permanent loss of lung function.

Chronic obstructive pulmonary disease (COPD), which is considered one of the representative lung diseases along with asthma, differs from asthma in that COPD involves irreversible airway obstruction. COPD is a disease caused by pathological changes in the bronchioles and lung parenchyma due to inflammation of the airways and lung parenchyma, and is characterized by bronchiolitis obliterans and emphysema. Types of COPD include chronic obstructive bronchitis, chronic bronchiolitis, and emphysema. Symptoms thereof are shortness of breath, cough, and phlegm, *etc.* The majority of chronic bronchitis patients are known to have COPD.

In addition, pulmonary fibrosis is a type of chronic interstitial lung disease that causes severe respiratory failure through pulmonary interstitial infiltration of inflammatory cells such as lymphocytes and macrophages, fibroblast proliferation, and pulmonary interstitial deposition of fibrous connective tissue. As pulmonary fibrosis progresses, various complications appear, including systemic hypoxia caused by decreased lung capacity and impaired oxygen diffusion, pulmonary hypertension caused by compression of pulmonary arteries and capillaries as fibrous tissue contracts, and heart failure caused by stagnation of blood in the right ventricle due to pulmonary circulation disorders, thrombosis, and pneumonia. Additionally, the incidence of lung cancer may be increased due to inflammation and fibrosis.

Most of these various lung diseases are associated with inflammation or fibrosis, and currently there is no known treatment for lung disease that can simultaneously improve these two conditions.

In response, the inventors of the present application have made efforts to effectively treat various lung diseases associated with inflammation or fibrosis without side effects, and have confirmed that umbilical cord blood-derived mesenchymal stem cells overexpressing CD47 exhibit anti-inflammatory and anti-fibrotic effects, completing the present disclosure.

### Detailed Description of the disclosure

### Technical Problem

The objective of the present disclosure is to provide a pharmaceutical composition for use in preventing or treating lung diseases which exhibits anti-inflammatory and anti-fibrotic effects.

Another objective of the present disclosure is to provide a method of treating lung disease including administering to a subject a composition containing mesenchymal stem cells expressing CD47 as an active ingredient.

Another objective of the present disclosure is to provide use of mesenchymal stem cells expressing CD47 in the production of therapeutic agents for use in the treatment of lung diseases.

### Technical Solution to Problem

In order to achieve these objectives, one aspect provides a pharmaceutical composition for use in preventing or treating lung disease containing mesenchymal stem cells expressing CD47 as an active ingredient.

The term "cluster of differentiation 47 (CD47)" is also called integrin associated protein (IAP) and refers to a cell surface glycoprotein which is bonded to ligand thrombospondin-1 (TSP-1) and signal-regulatory protein alpha (SIRPα) and is expressed in human cells. It is known that suppressing the expression of CD47 in tumor tissues has some cancer treatment effects by inducing phagocytosis of T cell-mediated adaptive immunity while targeting CTLA and PD-1. Activation of CD47 was found to be correlated with enhanced cancer cell proliferation through the P13K/Akt pathway. In addition to immune responses, CD47 is involved in a variety of cellular processes, including apoptosis, proliferation, adhesion, and migration.

The term "mesenchymal stem cells (MSCs)" may refer to stem cells present in cartilage, bone tissues, adipose tissue, and bone marrow stroma which are differentiated from mesoderm resulting from division of a fertilized egg, and may include mesenchymal stem cells of mammals, for example, animals including humans. Mesenchymal stem cells maintain stemness and self-renewal, have the ability to differentiate into various cells, including cartilage cells, osteoblasts, muscle cells, and adipocytes, and bone marrow cells, and may be extracted from bone marrow, adipose tissues, umbilical cord blood, synovial membrane, trabecular bone, muscle, infrapatellar fat pad, etc. Mesenchymal stem cells has the ability to regulate immunity including: suppression of the activity and proliferation of T lymphocytes and B lymphocytes; suppression of the activity of natural killer cells (NK cells); and regulation of the functions of dendritic cells and macrophages. Accordingly, mesenchymal stem cells amount to a cell capable of allotransplantation and xenotransplantation.

The mesenchymal stem cells may be genetically engineered to increase expression of CD47.

The term "genetic engineering" or "genetically engineered" may refer to the act of introducing one or more genetic modifications to a cell.

The increase in expression or overexpression refers to a higher level of expression of a protein compared to the expression of the same type of endogenous protein of a given non-genetically engineered parent cell (for example, wild type).

The genetically engineered mesenchymal stem cells may include a recombinant vector into which an exogenous gene encoding CD47 has been introduced.

In an embodiment, mesenchymal stem cells overexpressing CD47 may be produced using a viral vector into which an exogenous gene encoding CD47 has been introduced.

The exogenous gene may be expressed in an amount sufficient to increase the activity of CD47 compared to the parent cell in the mesenchymal stem cell or a host cell. The exogenous gene may be introduced into a mother cell through an expression vector. In some embodiments, the exogenous gene may be introduced into a mother cell in the form of a linear polynucleotide. In some embodiments, the exogenous gene may be expressed from an expression vector (for example, plasmid) *in vivo.* In some embodiments, for stable expression, the exogenous gene may be inserted into a genetic material (for example, chromosome) within a cell and expressed. In some embodiments, the exogenous gene may be appropriately regulated by an exogenous promoter operably linked to a gene. Enhancement of the expression level of the polynucleotide may result from modification by substitution or mutation in the expression regulatory sequence; introduction of a mutation in the polynucleotide sequence itself; replacement of the start codon; an increase in copy number by insertion into the chromosome or introduction through a vector; or a combination of these.

The term "operably linked" as used herein refers to a functional linkage between a regulatory sequence (for example, a promoter) and the polynucleotide sequence, whereby the regulatory sequence controls transcription and/or translation of the polynucleotide sequence. The regulatory sequence may be a strong promoter capable of increasing the expression level of the polynucleotide. The regulatory sequence may be a promoter from the genus *Corynebacterium* or other microorganisms. The promoter may be, for example, a trc promoter, a gap promoter, a tac promoter, a T7 promoter, a lac promoter, a trp promoter, a araBAD promoter, or a cj 1 to 7 promoter. The regulatory sequence may be, for example, a promoter sequence of a major gene in the lysine biosynthetic pathway in genus *Corynebacterium* microorganisms that has been modified to have higher promoter activity.

The term "vector" may refer to a polynucleotide construct containing the regulatory sequence and the nucleotide sequence of a gene so that the gene of interest can be expressed in a suitable host cell. Also, it may refer to a polynucleotide construct containing a nucleotide sequence capable of homologous recombination, which allows the change of the regulatory sequence of an endogenous gene in the host cell's genome or the insertion of a target gene expressible at a specific site in the genome by introducing the polynucleotide construct into the host cell. Therefore, the vector may additionally include a selection marker to confirm introduction into the host or chromosomal insertion. The selection marker may be a marker that confers a selectable phenotype, such as drug resistance, nutritional requirements, resistance to cytotoxic agents, or expression of a surface protein. In an environment treated with a selection agent, only cells expressing the selection marker survive or other expression traits are shown. Accordingly, transformed cells can be selected. The vectors may include viral vectors such as plasmid vectors, lentiviral vectors, cosmid vectors and bacteriophage vectors, adenovirus vectors, retroviral vectors and adeno-associated viral vectors. In addition, vectors that can be used as the recombinant vector may be those obtained by manipulating plasmids often used in the art (for example, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19, etc.), phages (for example, λgt4λB, λ-Charon, λ△z1, and M13, etc.), or virus (for example, SV40, etc.).

The recombinant vector may typically be constructed as a vector for cloning or a vector for expression. The expression vector may be selected from those that are commonly used in the art to express foreign proteins in plants, animals, or microorganisms. The recombinant vector may be constructed through various methods known in the art.

The recombinant vector may be constructed using prokaryotic cells or eukaryotic cells as hosts. For example, when the vector used is an expression vector and a prokaryotic cell is used as the host, the vector may include a strong promoter capable of advancing transcription (for example, a pLλ promoter, a CMV promoter, a trp promoter, a lac promoter, a tac promoter, a T7 promoter, etc.), a ribosome binding site for initiation of translation, and a transcription/translation termination sequence. In the case of using a eukaryotic cell as a host, the origin of replication operating in the eukaryotic cell included in the vector may include the f1 origin of replication, the SV40 origin of replication, the pMB1 origin of replication, the adeno origin of replication, the AAV origin of replication, and the BBV origin of replication, but is not limited thereto. Additionally, promoters derived from the genome of mammalian cells (for example, metallothionein promoter) or promoters derived from mammalian viruses (for example, adenovirus late promoter, vaccinia virus 7.5K promoter, SV40 promoter, cytomegalovirus promoter, and the tk promoter of HSV) may be used, and typically, a polyadenylation sequence may be used as the transcription termination sequence.

The recombinant cell may be obtained by introducing the recombinant vector into an appropriate host cell. The host cell may be any cell that is capable of stably and continuously cloning or expressing the recombinant vector and is known in the art, and prokaryotic cells include, for example, E. coliJM109, E. coliBL21, E. coli RR1, E. coli LE392, E. coli B, E. coli X 1776, E. coli W3110; strains of the genus *Bacillus,* such as *Bacillus* subtilis, *Bacillus* churrigensis; and enterobacteriaceae strains such as *Salmonella* typhimurium, *Serratia* marcescens, and various *Pseudomonas* species. For transformation into eukaryotic cells, yeast (Saccharomyces cerevisiae), insect cells, plant cells, and animal cells may be used as host cells, and examples thereof are Sp2/0, Chinese hamster ovary (CHO) K1, CHO DG44, PER.C6, W138, BHK, COS-7, 293, HepG2, Huh7, 3T3, RIN, and MDCK cell lines.

The mesenchymal stem cells may be derived from any one selected from the group consisting of umbilical cord, umbilical cord blood, placenta, bone marrow, adipose tissue, muscle, amniotic fluid, amniotic membrane, and dental pulp.

The term "isolated cell", for example "isolated mesenchymal stem cell" or "derived cell", for example "derived mesenchymal stem cell", refers to a cell that is substantially separated from the tissue from which the cell originates, for example, umbilical cord blood.

The umbilical cord may refer to a cord that connects the mother and the abdomen so that the mammalian fetus can grow in the placenta, and generally, may refer to a tissue consisting of three blood vessels surrounded by Wharton's jelly, namely, two umbilical arteries and one umbilical vein, and is also called umbilicus.

The umbilical cord blood may refer to blood collected from the umbilical cord vein that connects the placenta and fetus of a mammal. The umbilical cord blood may be easily collected from the donor's umbilical cord vein at the time of birth. Specifically, in the case of normal vaginal delivery, the sample can be collected from the umbilical vein which is expelled from the uterus while the placenta still remain therein after birth. In the case of cesarean section, the sample can be collected from the umbilical vein while the placenta is expelled outside the uterus after birth.

In an embodiment, the mesenchymal stem cells may be derived from umbilical cord blood.

20% to 99% of a population of the mesenchymal stem cells may express CD47.

The mesenchymal stem cells may have at least 20%, 25%, 30%, 35%, 40%, 45%, 50%, 55%, 60%, 65%, 70%, 75%, 80%, 85%, 90%, 95%, 98% or about 99%, or 30% to 100%, 30% to 99%, 30% to 98%, 30% to 97%, 40% to 99%, 50% to 99%, 60% to 99%, 70% to 99%, 80% to 99% or 90% to 99% of the cell population expressing CD47.

In an embodiment, it was confirmed that in the case of the control mesenchymal stem cells, about 29% of cell population expressed CD47, and in the case of the mesenchymal stem cell including the recombinant vector into which an exogenous gene encoding CD47 has been introduced, about 96% of the cell population expressed CD47.

Specifically, 20% to 85%, 20% to 70%, 20% to 60%, and 25% to 50% of the non-genetically modified mesenchymal stem cell population may express CD47. 80% to 100%, 85% to 99%, or 90% to 99% of the genetically engineered mesenchymal stem cell population may express CD47.

The mesenchymal stem cells may be i) positive for one or more markers selected from the group consisting of CD90, CD105, CD73, and CD166, and ii) negative for CD14 or HLA-DR marker.

The term "positive or +" refers to a case where, in relation to a mesenchymal stem cell-specific marker, the marker is present in a greater amount or at a higher concentration compared to other reference cells. In other words, a cell is positive for a marker in the case where the marker can be used to distinguish the cell from one or more other cell types because the marker is present inside or on the surface of a cell. Also, it may also indicate that the cell is expressing the marker in an amount sufficient to produce a signal greater than the background value, for example, a signal of a cytometry device. For example, a cell may be detectably labeled with a CD90-specific antibody, and in the case where the signal from this antibody is detectably greater than the control (for example, background value), the cell is "CD90⁺."

The term "negative or -" refers to a case where even using an antibody specific for a particular cellular marker, that marker cannot be detected compared to background values. For example, in the case where a cell cannot be detectably labeled with an antibody specific for CD14, the cell is "CD14".

In an embodiment, it was confirmed that in the case of the genetically engineered mesenchymal stem cell which overexpresses CD47 and the non-genetically engineered mesenchymal stem cell, at least 98% of each of the cell populations expressed CD90, CD105, CD73, and CD166 markers, and not more than 1% thereof expressed CD14 and HLA-DR markers. Therefore, it was confirmed that the genetically engineered CD47-overexpressing mesenchymal stem cells also retain mesenchymal stem cell-specific characteristics.

The mesenchymal stem cells may be positive for one or more markers selected from the group consisting of CD9, CD13, CD29, CD44, CD73, CD90, CD105, CD166, and HLA-abc.

The mesenchymal stem cells may be negative for one or more markers selected from the group consisting of CD2, CD3, CD4, CD5, CD7, CD8, CD14, CD15, CD16, CD19, CD20, CD22, CD33, CD36, CD38, CD61, CD62E, CD133, and HLA-DR.

The mesenchymal stem cells may exhibit reduced immunogenicity.

In an embodiment, it was confirmed that when CD47-overexpressing mesenchymal stem cells are co-cultured with PBMCs or NK cells, the cell death effect caused by activated PBMCs and NK cells is reduced. Therefore, when the CD47-overexpressing mesenchymal stem cells are administered, the immune response caused by activated PBMCs or NK cells can be avoided even if they are not autologous, and thus, the *in vivo* survival rate of the administered cells may be increased.

The lung disease may be asthma, pneumonia, chronic obstructive pulmonary disease, bronchitis, emphysema, pulmonary hypertension, or pulmonary fibrosis, but is not limited thereto.

The mesenchymal stem cells may reduce the inflammatory response in lung tissues.

The mesenchymal stem cells may decrease M1 macrophages and increase M2 macrophages.

M1 macrophages induce an inflammatory response, and M2 macrophages reduce the inflammatory response and repair damaged tissues.

In an embodiment, as a result of intravenous administration of CD47-overexpressing mesenchymal stem cells to an asthma mouse model, it was confirmed that the CD47-overexpressing mesenchymal stem cells migrated well to lung tissue, and the expression of a marker that is specific for M1 macrophages, which induce an inflammatory response in lung tissues was reduced, and the expression of a marker that is specific for M2 macrophages, which repair damaged tissues, was increased. In addition, it was confirmed that the expression of inflammatory cytokines and the number of neutrophils in the bronchoalveolar lavage fluid (BALF) of an asthma mouse model were reduced.

Therefore, the CD47-overexpressing mesenchymal stem cells can be used for the prevention or treatment of diseases associated with inflammatory responses in lung tissues, including asthma.

Mesenchymal stem cells overexpressing CD47 may reduce the expression of α-smooth muscle actin (α-SMA) and matrix metalloproteinase-13 (MMP-13).

In an embodiment, as a result of co-culturing CD47-overexpressing mesenchymal stem cells and human lung fibroblasts (HPF), it can be confirmed that the expression of fibrosis-specific markers is reduced.

Therefore, the CD47-overexpressing mesenchymal stem cells may be used to prevent or treat diseases associated with fibrosis of lung tissues, including fibrosis.

Another aspect provides a pharmaceutical composition for use in preventing or treating lung disease including the CD47-overexpressing mesenchymal stem cell culture medium as an active ingredient.

The CD47-overexpressing mesenchymal stem cell culture medium may be obtained by subculturing the CD47-overexpressing mesenchymal stem cells in a suitable medium and then filtering the culture medium.

Another aspect provides a cell therapy agent or a pharmaceutical composition including the CD47-overexpressing mesenchymal stem cells or a cell population thereof as an active ingredient.

The cell therapy agent or the pharmaceutical composition may be for use in preventing or treating lung disease.

Another aspect provides use of CD47-overexpressing mesenchymal stem cells or a cell population thereof for the manufacture of a medicament.

In an embodiment, the use may be use of mesenchymal stem cells expressing CD47 for the manufacture of a therapeutic agent for treatment of lung diseases.

Another aspect provides a method of treating lung disease including administering the CD47-overexpressing mesenchymal stem cells or a cell population thereof to a subject in a therapeutically effective or pharmaceutically effective amount.

The cell therapy agent or the pharmaceutical composition may be administered to a patient in a therapeutically effective or pharmaceutically effective amount.

The term "treatment" refers to or includes alleviating, inhibiting the progression of, or preventing a disease, a disorder, or a condition, or one or more symptoms thereof, and the term "active ingredient" refers to a cell population or composition effective in alleviating, inhibiting the progression of, or preventing a disease, a disorder, or a condition, or one or more symptoms thereof.

The terms "administering," "introducing," and "implanting" are used interchangeably and may refer to the placement of the composition according to an embodiment into a subject by a method or route that results in at least partial localization thereof to a target site. It may be administered via any suitable route t which delivers a cell of the composition according to an embodiment or at least a portion of the cell component to a target site within a living subject. The survival period of cells after administration to a subject may be as short as several hours, for example, 24 hours to several days or as long as several years. The pharmaceutical composition may be administered to a patient in a therapeutically effective or pharmaceutically effective amount.

The term "therapeutically effective amount" or "pharmaceutically effective amount" refers to an amount of a cell population or composition effective in preventing or treating a lung disease, sufficient to treat the disease at a reasonable benefit/risk ratio applicable to medical treatment while not causing side effects. The level of the effective amount may be determined according to factors including the patient's health status, type and severity of the disease, activity of the drug, sensitivity to the drug, an administration method, an administration time, the route of administration and the excretion rate, treatment period, drugs combined or used simultaneously, and other factors well known in the medical field.

The pharmaceutical composition may be administered as an individual therapeutic agent or in combination with other therapeutic agents, may be administered sequentially or simultaneously with conventional therapeutic agents, and may be administered singly or multiple times. Considering all of these factors, it is important to administer an amount that may achieve the maximum effect with the minimum amount without side effects, which may be easily determined by a person skilled in the art.

Specifically, the effective amount of the CD47-overexpressing mesenchymal stem cells in the pharmaceutical composition may vary depending on the patient's age, gender, and weight, and generally, about 1×10⁶ to 1×10⁷ cells per body weight (kg) may be administered once or repeatedly. As the effective amount may increase or decrease depending on the route of administration, severity of disease, gender, weight, age, etc., and accordingly, its range is not limited thereto.

Redundant description is omitted in consideration of the complexity of the present specification, and terms not defined otherwise in this specification have meanings commonly used in the technical field to which the present disclosure pertains.

### Advantageous Effects of Disclosure

According to one aspect, mesenchymal stem cells overexpressing CD47 show a high survival rate and ability to migrate to lung tissues *in vivo* even when they are not autologous, reduce the inflammatory response in lung tissues, and exhibit the effect of suppressing fibrosis. Therefore, such mesenchymal stem cells can be widely used for the prevention or treatment of various lung diseases associated with lung inflammation or fibrosis.

### Brief Description of Drawings

FIG. 1 shows the results of confirming the CD47 expression rates of CD47-overexpressing mesenchymal stem cells (MSC O.E.) according to one aspect and control mesenchymal stem cells (MSC V.C.).
FIG. 2 shows the results of comparing the expression levels of mesenchymal stem cell-specific markers of CD47-overexpressing mesenchymal stem cells (MSC O.E.) according to one aspect and control mesenchymal stem cells (MSC V.C.).
FIG. 3 shows the results of comparing the inflammation improvement effects in lung tissues after administering CD47-overexpressing mesenchymal stem cells (MSC O.E.) according to one aspect and control mesenchymal stem cells (MSC V.C) to an asthma mouse model.
FIG. 4 shows images of immunocytochemical staining to confirm changes in the expression of CD11b, which is an M1 macrophage specific marker, in lung tissues after administering CD47-overexpressing mesenchymal stem cells (MSC O.E.) according to one aspect and control mesenchymal stem cells (MSC V.C) to an asthma mouse model.
FIG. 5 shows the results of measuring the number of cells expressing CD11b, an M1 macrophage-specific marker, in lung tissues after administering CD47-overexpressing mesenchymal stem cells (MSC O.E.) according to one aspect and control mesenchymal stem cells (MSC V.C) to an asthma mouse model.
FIG. 6 shows images of immunocytochemical staining to confirm changes in the expression of CD163, which is an M2 macrophage specific marker, in lung tissues after administering CD47-overexpressing mesenchymal stem cells (MSC O.E.) according to one aspect and control mesenchymal stem cells (MSC V.C) to an asthma mouse model.
FIG. 7 shows the results of measuring the number of cells expressing CD163, an M2 macrophage-specific marker, in lung tissues after administering CD47-overexpressing mesenchymal stem cells (MSC O.E.) according to one aspect and control mesenchymal stem cells (MSC V.C) to an asthma mouse model.
FIG. 8 shows images of immunocytochemical staining to confirm migration to lung tissues after intravenously administering CD47-overexpressing mesenchymal stem cells (MSC O.E.) according to one aspect and control mesenchymal stem cells (MSC V.C) to an asthma mouse model.
FIG. 9 shows the results of measuring the number of CD47-overexpressing mesenchymal stem cells migrated to lung tissues after intravenously administering CD47-overexpressing mesenchymal stem cells (MSC O.E.) according to one aspect and control mesenchymal stem cells (MSC V.C) to an asthma mouse model.
FIG. 10 shows the change in the expression of inflammatory cytokines in bronchoalveolar lavage fluid (BALF) after administering CD47-overexpressing mesenchymal stem cells (MSC O.E.) according to one aspect and control mesenchymal stem cells (MSC V.C) to an asthma model.
FIG. 11 shows the results of confirming changes in the number of neutrophil cells in BALF after administering CD47-overexpressing mesenchymal stem cells (MSC O.E.) according to one aspect and control mesenchymal stem cells (MSC V.C) to an asthma model.
FIG. 12 shows the results of confirming survival changes in mesenchymal stem cells through immunocytochemical staining, after CD47-overexpressing mesenchymal stem cells (MSC O.E.) according to one aspect and control mesenchymal stem cells (MSC V.C) were each co-cultured with activated peripheral blood mononuclear cells (PBMC).
FIG. 13 shows the results of measuring the survival rate of mesenchymal stem cells after co-culturing each of CD47-overexpressing mesenchymal stem cells (MSC O.E.) according to one aspect and control mesenchymal stem cells (MSC V.C) with PBMC.
FIG. 14 shows the results of confirming survival changes in mesenchymal stem cells through immunocytochemical staining, after CD47-overexpressing mesenchymal stem cells (MSC O.E.) according to one aspect and control mesenchymal stem cells (MSC V.C) were each co-cultured with activated natural killer cells (NK cells).
FIG. 15 shows the results of measuring the survival rate of mesenchymal stem cells after co-culturing each of CD47-overexpressing mesenchymal stem cells (MSC O.E.) according to one aspect and control mesenchymal stem cells (MSC V.C) with activated NK cells.
FIG. 16 shows the results of confirming the change in expression level of fibrosis-specific markers after co-culturing CD47-overexpressing mesenchymal stem cells (MSC O.E.) according to one aspect and control mesenchymal stem cells (MSC V.C) with human lung fibroblasts (HPF).

### Mode of Disclosure

Hereinafter, the present disclosure will be explained in more detail by examples. However, these examples are for illustrative purposes only and the scope of the present disclosure is not limited by these examples.

### Preparation Example 1. Isolation of mesenchymal stem cells from umbilical cord blood

With the consent of a healthy mother, donated umbilical cord blood obtained from the umbilical vein donated during delivery was centrifuged using a Ficoll-Hypaque gradient to obtain mononuclear cells, which were then cultured in mesenchymal stem cell culture medium to obtain umbilical cord blood-derived mesenchymal stem cells.

### Preparation Example 2. Preparation of CD47-overexpressing mesenchymal stem cells

To produce CD47-overexpressing lentivirus, the CD47 open reading frame (ORF) sequence was cloned into plenti-suCMV-RSV-GFP vector using the EZ-fusion HT cloning kit (#EZ015, Enzynomics). The plenti-suCMV-RSV-GFP-CD47, pMD2.G, and psPAX2 vectors were transfected into 293T cells to package the lentivirus. The medium containing virus particles was collected and processed into mesenchymal stem cells for transduction. The cells were treated with the antibiotic puromycin (#ant-pr-1, Invivogen) for 2 days and only transduced stem cells were selected and cultured to prepare CD47-overexpressing mesenchymal stem cells (overexpression, O.E.). Control mesenchymal stem cells (Vector control, V.C.) for analysis of CD47 expression level and function were prepared using the backbone vector plenti-suCMV-RSV-GFP through lentiviral packaging and transfection and antibiotic selection in the same manner as used to prepare CD47-overexpressing mesenchymal stem cells.

### Example 1. Analysis of characteristics of CD47-overexpressing mesenchymal stem cells

To analyze the characteristics of the CD47-overexpressing mesenchymal stem cells prepared in Preparation Example 2, Western blot and flow cytometry were performed as follows.

To describe the Western blot method in more detail, control mesenchymal stem cells and CD47-overexpressing mesenchymal stem cells were each treated with RIPA lysis buffer (#78440, Thermo Scientific) and caused to react on ice for 30 minutes to disrupt the cells. After centrifuging this cell lysate, only the supernatant was collected and quantified using the BCA protein assay kit (#23225, Thermo Scientific). 10 µg of protein extract was separated using 10% SDS-PAGE and then transferred to a membrane. After a reaction was caused to occur using a blocking buffer (0.1% Tween 20, 5% BSA in tris buffered saline) at room temperature for 1 hour, the cells were treated with primary antibodies and a reaction was caused to occur at 4°C overnight. The primary antibodies used were CD47 (#MA5-11895, Thermo Scientific) and b-actin (#A5441, Sigma). After washing three times with TBST, a secondary antibody was reacted at room temperature for 1 hour, and after washing, the protein band was analyzed using the ChemiDoc MP System (Bio-rad).

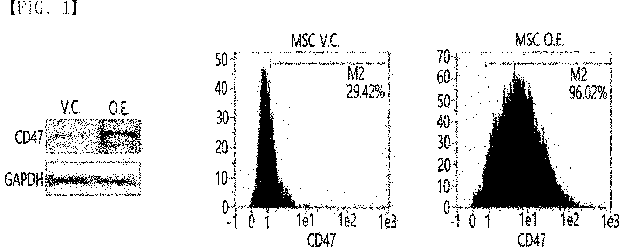

Flow cytometry was performed to confirm CD47 overexpression on the cell surface. More specifically, for flow cytometric analysis of CD47 protein expression, cells were stained with PE mouse anti-human CD47 (#556046, BD Biosciences) for 15 minutes at room temperature. The corresponding PE Mouse IgG1, k Isotype (#555749, BD Biosciences) was used as a control. After washing once with PBS, the cells were fixed with 1% PFA and the protein expression level was analyzed on a MACS Quant instrument (MACS Quantify). Results are shown in FIG. 1.

As shown in FIG. 1, it was confirmed that the amount of CD47 protein expression was significantly increased in the CD47-overexpressing stem cell population (O.E.) compared to the control mesenchymal stem cell population (MSC V.C.). Specifically, the number of cells expressing CD47 was 29.42% in control I mesenchymal stem cells, but increased to 96.02% in CD47-overexpressing mesenchymal stem cells.

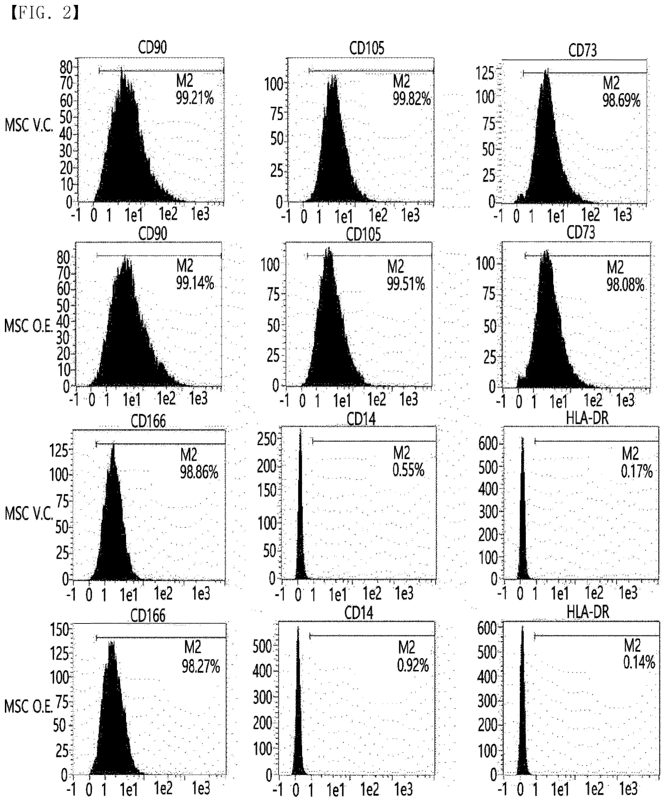

In addition, the expression levels of mesenchymal stem cell-specific markers in the control mesenchymal stem cell population (MSC V.C.) and the CD47-overexpressing stem cell population (MSC O.E.) were compared through flow cytometry using the method described above. Results are shown in FIG. 2.

As shown in FIG. 2, it was confirmed that, in the control mesenchymal stem cell population (MSC V.C.) and the CD47-overexpressing stem cell population (MSC O.E.), the number of cells expressing the mesenchymal stem cell-specific markers CD90, CD105, CD73, and CD166 was 98% or more, and the number of cells expressing CD14 and HLA-DR was less than 1%. As a result, it was found that the induction of overexpression of CD47 did not affect the change in the specific phenotype of mesenchymal stem cells.

### Example 2. Confirmation of effects of administration of CD47-overexpressing mesenchymal stem cells to an asthma animal model (in-vivo) on lung tissues

C57BL/6J female mice (6 weeks old) were purchased from Orient Bio Inc. and acclimatized for 1 week. To induce asthma, 100 µg of Ovalbumin (OVA; Sigma-Aldrich) and 2 mg of Alum (Sigma-Aldrich) were intraperitoneally administered to mice on day 0 and day 7 (a total of two times). Thereafter, mesenchymal stem cells overexpressing CD47 prepared in Preparation Example 2 were intraperitoneally administered on day 20 (5 × 10⁵ cells/100 µL/mouse). On day 21 to day 23, mice were anesthetized and 50 µg of OVA (30 µL/mouse) was administered intranasally. On day 24, all mice were sacrificed, and bronchoalveolar lavage fluid (BALF) was obtained therefrom with 2 ml of saline by using an intravenous catheter.

### Example 2.1 Confirmation of inflammatory changes in lung tissues

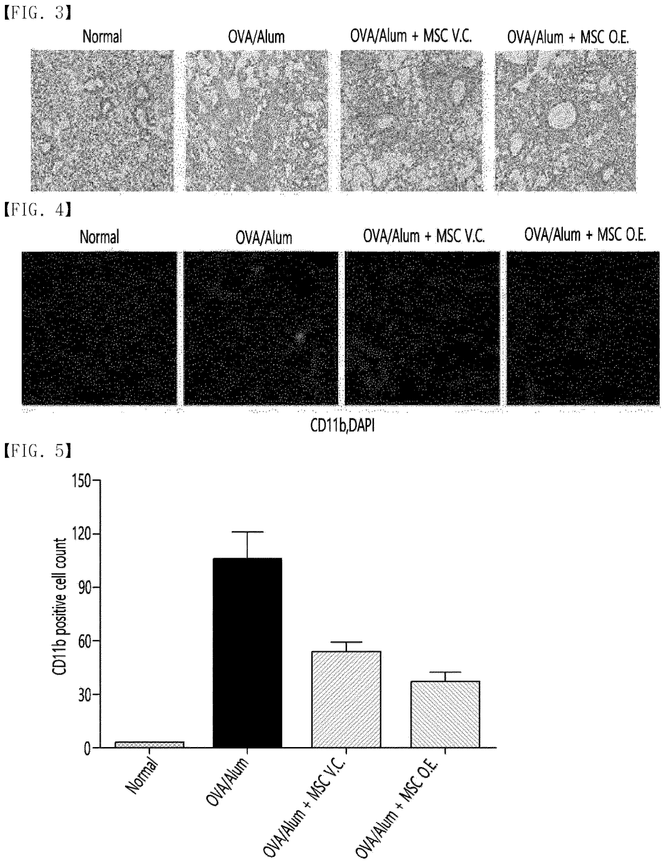

The obtained lung tissue sections were stained with Hematoxyling and Eosin (H&E) and tissue analysis was performed. Results are shown in FIG. 3.

As shown in FIG. 3, although inflammation in lung tissues was alleviated in the control mesenchymal stem cell (MSC V.C.) administered group, but the extent of inflammation improvement in the CD47-overexpressing mesenchymal stem cells (MSC O.E.) administered group was substantially greater compared to the control mesenchymal stem cell administered group.

### Example 2.2 Confirmation of changes in expression of M1 macrophage specific markers in lung tissues

Lung tissue sections were immunocytochemically stained with Integrin Alpha M (CD11b; Abbexa) at a concentration of 1:40, and Cy3 Rabbit anti-mouse IgG1 (Jackson ImmunoResearch Europe Ltd.) was attached as a secondary antibody to confirm the expression level of CD11b. The nuclei of cells in lung tissues were stained with 4',6-diamidino-2-phenylindole (DAPI). Results are shown in FIGS. 4 and 5.

As shown in FIGS. 4 and 5, when CD47-overexpressing mesenchymal stem cells (MSC O.E.) were administered to an asthma mouse model, it was confirmed that the expression level of CD11b, which is the M1 macrophage-specific marker, was reduced compared to the control mesenchymal stem cell (MSC V.C.).

### Example 2.3 Confirmation of changes in expression of M2 macrophage-specific markers in lung tissues

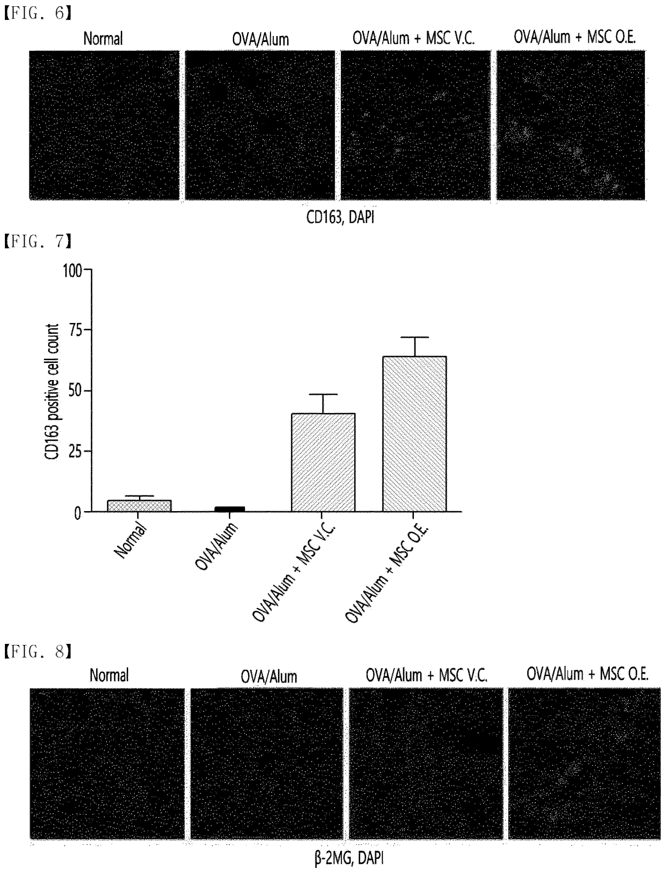

Lung tissue sections were immunocytochemically stained with scavenger receptor cysteine-rich type 1 protein M130 (CD163; Abbexa) at a concentration of 1:30, and Alexa 488 rabbit anti-mouse IgG1 (Jackson ImmunoResearch Europe Ltd.) was attached as a secondary antibody to confirm the expression level of CD163. Nuclei of cells in lung tissue were stained with DAPI. Results are shown in FIGS. 6 and 7.

As shown in FIGS. 6 and 7, when CD47-overexpressing mesenchymal stem cells (MSC O.E.) were administered to an asthma mouse model, it was confirmed that the expression level of CD163, which is the M2 macrophage-specific marker, was reduced compared to the control mesenchymal stem cell (MSC V.C.).

### Example 2.4 Confirmation of the number of cells migrated to lung tissues after intravenous administration

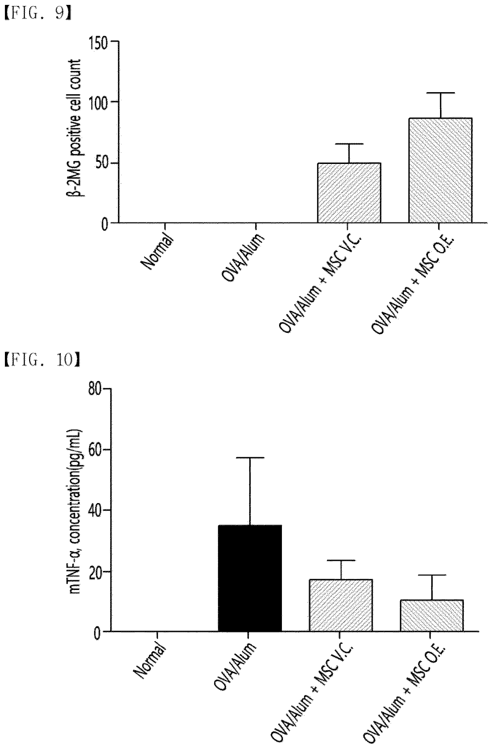

Lung tissue sections were immunocytochemically stained with anti-beta 2 microglobulin antibody (β-2MG; Abcam) at a concentration of 1:300, and Alexa 488 rabbit anti-mouse IgG1 (Jackson ImmunoResearch Europe Ltd.) was attached as a secondary antibody to confirm the expression level of β-2MG. Nuclei of cells in lung tissues were stained with DAPI. Results are shown in FIGS. 8 and 9.

As shown in FIGS. 8 and 9, when CD47-overexpressing mesenchymal stem cells (MSC O.E.) were administered to an asthma mouse model, it was confirmed that the expression level of β-2MG in lung tissues was higher compared to the control mesenchymal stem cell (MSC V.C.) administered group. Through these, it was found that the number of mesenchymal stem cells that migrated to lung tissue after intravenous administration was greater in CD47-overexpressing mesenchymal stem cells (MSC O.E.).

### Example 3. Confirmation of effects of administration of CD47-overexpressing mesenchymal stem cells to an asthma animal model (in-vivo) on BALF

### Example 3.1 Confirmation of changes in expression of inflammatory cytokines in BALF

The BALF obtained in Example 2 was centrifuged at 4°C and 1000 rpm for 10 minutes, and in the separated supernatant, mouse tumor necrosis factor-α (mTNF-α; R&D systems) was quantified using an enzyme-linked immunosorbent assay (ELISA). Results are shown in FIG. 10.

As shown in FIG. 10, when CD47-overexpressing mesenchymal stem cells (MSC O.E.) were administered to an asthma mouse model, it was confirmed that the secretion amount of mTNF-α, which is an inflammatory cytokine, was the lowest compared to the control mesenchymal stem cell (MSC V.C.) administered group.

### Example 3.2 Confirmation of reduction in the number of neutrophil cells in BALF

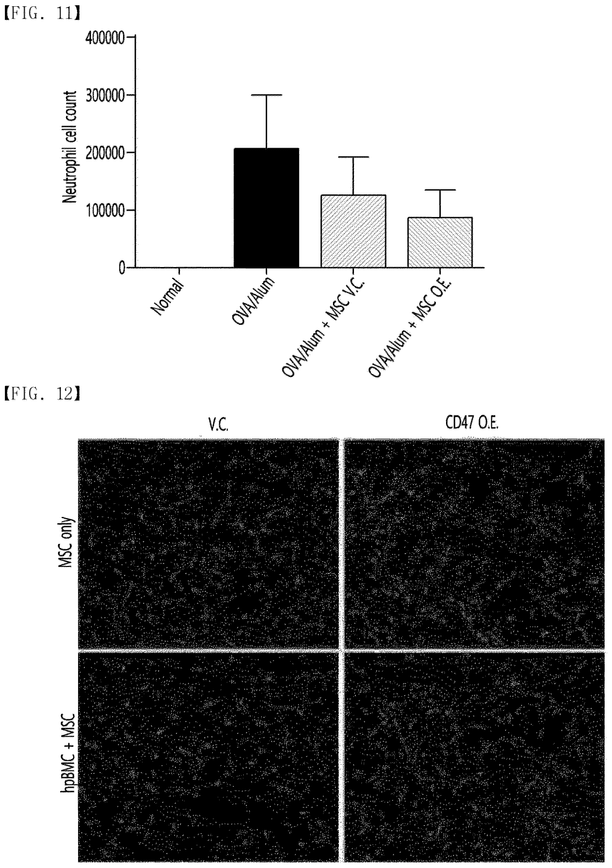

BALF obtained in Example 2 was centrifuged and settled cells were stained with Diff-Quik, and the number of neutrophil cells was measured. Results are shown in FIG. 11.

As shown in FIG. 11, the number of neutrophil cells which had been increased in the asthma-induced mouse model, was decreased in the mesenchymal stem cell administration group, and especially, the decrease in the number of neutrophil cells in the CD47-overexpressing mesenchymal stem cells (MSC O.E.) was the greatest.

### Example 4. Confirmation of immunomodulatory effect of CD47-overexpressing mesenchymal stem cells (in-vitro)

### Example 4.1 Confirmation of immunomodulatory effect through co-culture with PBMC

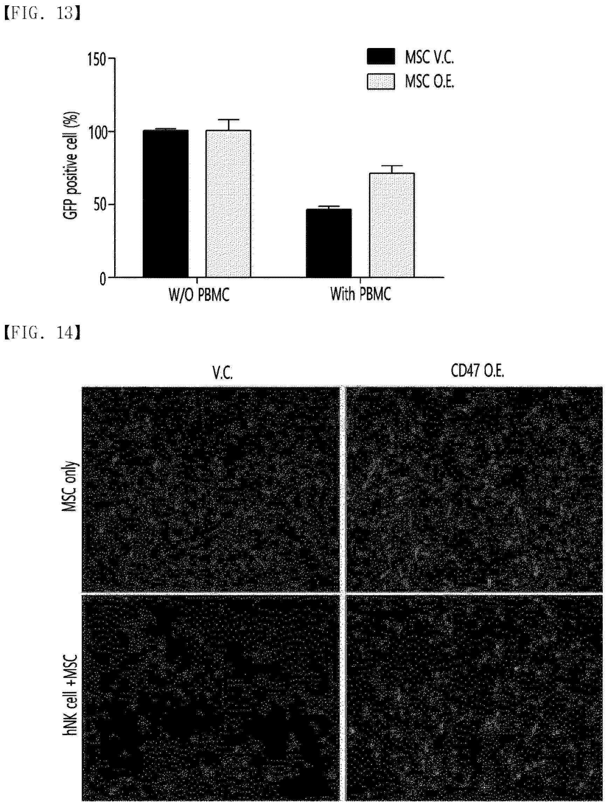

Mesenchymal stem cells for each condition were co-cultured with human peripheral blood mononuclear cells (PBMC; AllCells) which had been activated with 10 ng/mL of recombinant human interleukin 2 (hIL-2; miltenyi Biotec) and 5 µg/mL of phytohemagglutinin-L (PHA; Roche). 24 hours later, GFP fluorescence expressed in mesenchymal stem cells was observed and counted using a fluorescence microscope, and results thereof are shown in FIGS. 12 and 13.

As shown in FIGS. 12 and 13, in the case of CD47-overexpressing mesenchymal stem cells (MSC O.E.), 70.3% of cells survived, and in the case of the control mesenchymal stem cells (MSC V.C.), 44.5% of cells survived. As a result, it can be seen that CD47-overexpressing mesenchymal stem cells (MSC O.E.) were more resistant to cell death caused by activated PBMC compared to control mesenchymal stem cells (MSC V.C.).

### Example 4.2 Confirmation of immunomodulatory effect through co-culture with NK cells

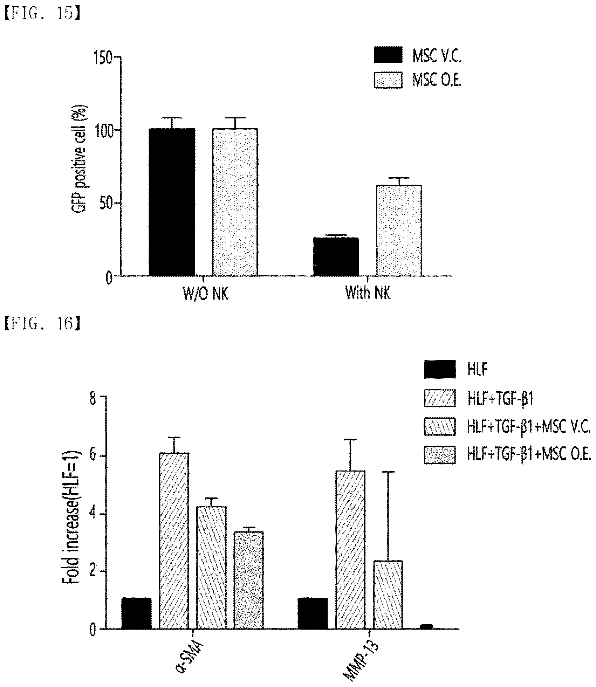

Mesenchymal stem cells for each condition were co-cultured with human natural killer cells (NK cells; Lonza) activated with 50 IU of IL-2 and 5% human serum (HS; Sigma-aldrich) for 2 weeks. Three hours later, GFP fluorescence expressed in mesenchymal stem cells was observed and counted using a fluorescence microscope, and results thereof are shown in FIGS. 14 and 15.

As shown in FIGS. 14 and 15, in the case of CD47-overexpressing mesenchymal stem cells (MSC O.E.), 63.8% of cells survived, and in the case of the control mesenchymal stem cells (MSC V.C.), 26.1% of cells survived. As a result, it can be seen that CD47-overexpressing mesenchymal stem cells (MSC O.E.) were more resistant to cell death caused by activated NK cells compared to control mesenchymal stem cells (MSC V.C.).

### Example 5. Confirmation of anti-fibrotic effect of CD47-overexpressing mesenchymal stem cells (in-vitro)

Human primary lung fibroblast (HLF; ATCC) was treated with 5 ng/mL of fibrosis-inducing substance TGF-β1 (R&D systems) for 24 hours, and then co-cultured with an equal amount of mesenchymal stem cells by using a transwell (BD Falcon) with a 1 µm pore size. After completion of co-culture, RNA of HLF was obtained using trizol (Invitrogen), and cDNA was synthesized using amfirivert cDNA synthesis platinum master mix (GenDEPOT). To confirm the expression levels of alpha-smooth muscle actin (α-SMA) and matrix metalloproteinase-13 (MMP-13), which are representative fibrosis markers, from synthesized cDNA, quantitative real time polymerase chain reaction (qPCR) was performed using LightCycler 480 (Roche). Results are shown in FIG. 16. Primer sequences used in PCR are as listed in Table 1 below.

**Table 1**

| Gene name | Nucleotide sequence | Sequence number |
|---|---|---|
| α-SMA (FORWARD) | 5'TTG CCT GAT GGG CAA GTG AT 3' | 1 |
| α-SMA (REVERSE) | 5'TAC ATA GTG GTG CCC CCT GA 3' | 2 |
| MMP-13 (FORWARD) | 5'TCC TCT TCT TGA GCT GGA CTC ATT 3' | 3 |
| MMP-13 (REVERSE) | 5'CGC TCT GCA AAC TGG AGG TC 3' | 4 |

As shown in FIG. 16, the expressions of α-SMA and MMP-13, which are representative fibrosis markers, were most reduced under HLF conditions of the co-culture with CD47-overexpressing mesenchymal stem cells (MSC O.E.), compared to that of control mesenchymal stem cells (MSC O.E.).

## Claims

1. A pharmaceutical composition for use in preventing or treating lung disease, comprising mesenchymal stem cells expressing CD47 as an active ingredient.

2. The pharmaceutical composition for use in preventing or treating lung disease of claim 1, wherein the mesenchymal stem cells are genetically engineered to increase expression of CD47.

3. The pharmaceutical composition for use in preventing or treating lung disease of claim 2, wherein the genetically engineered mesenchymal stem cells comprise a recombinant vector into which a gene encoding CD47 has been introduced.

4. The pharmaceutical composition for use in preventing or treating lung disease of claim 1, wherein 20% to 99% of a population of the mesenchymal stem cells express CD47.

5. The pharmaceutical composition for use in preventing or treating lung disease of claim 1, wherein the mesenchymal stem cells are
i) positive for one or more markers selected from the group consisting of CD90, CD105, CD73, and CD166, and
ii) negative for CD14 marker or HLA-DR marker.

6. The pharmaceutical composition for use in preventing or treating lung disease of claim 1, wherein the mesenchymal stem cells are derived from any one selected from the group consisting of umbilical cord, umbilical cord blood, placenta, bone marrow, adipose tissue, muscle, amniotic fluid, amniotic membrane, and dental pulp.

7. The pharmaceutical composition for use in preventing or treating lung disease of claim 1, wherein the mesenchymal stem cells exhibit reduced immunogenicity.

8. The pharmaceutical composition for use in preventing or treating lung disease of claim 1, wherein the mesenchymal stem cells reduce the inflammatory response of lung tissues.

9. The pharmaceutical composition for use in preventing or treating lung disease of claim 1, wherein the mesenchymal stem cells expressing CD47 decrease M1 macrophages and increase M2 macrophages.

10. The pharmaceutical composition for use in preventing or treating lung disease of claim 1, wherein the mesenchymal stem cells expressing CD47 reduce the expression of α-smooth muscle actin (α-SMA) and matrix metalloproteinase-13 (MMP-13).

11. The pharmaceutical composition for use in preventing or treating lung disease of claim 1, wherein the lung disease is asthma, pneumonia, chronic obstructive pulmonary disease, bronchitis, emphysema, pulmonary hypertension, or pulmonary fibrosis.

12. A method of treating lung disease, comprising administering, to a subject, a composition including mesenchymal stem cells expressing CD47 as an active ingredient.

13. Use of mesenchymal stem cells expressing CD47 for the manufacture of a therapeutic agent for the treatment of lung disease.
